(19) Europäisches Patentamt
European Patent Office
Office européen des brevets

(11) Publication number: 0 136 914
A2

# (12) EUROPEAN PATENT APPLICATION

(21) Application number: 84306729.9

(22) Date of filing: 03.10.84

(51) Int. Cl.[4]: A 61 K 7/08

(30) Priority: 04.10.83 US 538869

(43) Date of publication of application:
10.04.85 Bulletin 85/15

(84) Designated Contracting States:
AT BE CH DE FR IT LI LU NL SE

(71) Applicant: THE PROCTER & GAMBLE COMPANY
301 East Sixth Street
Cincinnati Ohio 45201(US)

(72) Inventor: Barford, Brian Dale
8002 Quail Hollow Court
West Chester Ohio 45069(US)

(72) Inventor: Cothran, Philip Earl
6941 Vern Drive
Cincinnati Ohio 45239(US)

(74) Representative: Brooks, Maxim Courtney et al,
Procter & Gamble (NTC) Limited Whitley Road
Longbenton
Newcastle-upon-Tyne NE12 9TS(GB)

## (54) Shampoo compositions.

(57) Antidandruff hair care compositions containing agglomerates of the antidandruff agent.

EP 0 136 914 A2

Croydon Printing Company Ltd.

0136914

# SHAMPOO COMPOSITIONS

BRIAN D. BARFORD
PHILIP E. COTHRAN

## BACKGROUND OF THE INVENTION

The present invention relates to antidandruff hair care compositions containing a water insoluble particulate antidandruff agent such as zinc pyridinethione in the form of agglomerates of smaller particles, at least 20% of the agglomerates being greater than 5 microns, equivalent spherical diameter, and the compositions being substantially free of polymeric and clay type suspending agents.

Zinc pyridinethione salts are well known in the art, as evidenced by U.S. Patent 2,809,971, October 15, 1957 to Bernstein and Losee. Furthermore, the use of such salts in detergents and shampoo compositions is disclosed in such references as U.S. Patent 3,236,733, February 11, 1966 to Karsten, et al; U.S. Patent 3,412,033, November 19, 1968 to Karsten and Taylor; U.S. Patent 3,580,853, May 25, 1971 to Parran; U.S. Patent 4,033,895, July 5, 1977 to Gerstein, British Patent 1,202,716, August 19, 1970 to Gerstein, et al; Japanese Patent 2,012,207, July 21, 1975 to Kao Soap KK; Japanese Patent 3,097,010, February 7, 1977 to Takeda Chemical Inc. II; Japanese Patent 6,061,308, October 25, 1979 to Shiseido KK; and European Patent Application 34,486, February 1981 to Winkler.

Other particulate antidandruff agents include sulfur and selenium sulfide. The use of these materials in detergent and shampoo compositions is shown in U.S. Patent 3,476,489, November 4, 1969 to Mees et al and British Patent 1,051,268, December 14, 1966 to Colgate-Palmolive Company, respectively.

Although many particulate antidandruff based hair care compositions have been developed, those products

have oftentimes suffered from inadequate deposition of the active ingredient and stability problems. In order to alleviate some of these problems, critical suspending media and particularly-shaped particles have been developed, the previously mentioned Winkler application and U.S. Patent 4,323,683, April 6, 1982 to Bolich, Jr., et al. However, even with these advances, optimal performance has not been achieved.

The present invention provides antidandruff hair care compositions which result in effective antidandruff agent deposition on the scalp. The enhanced deposition of the present compositions is believed to result from the larger particle size given by the agglomerates plus the lack of deposition interfering polymeric and clay agents. The above-described British Patent disclosing selenium sulfide compositions suggests the elimination of bentonite and sodium carboxymethylcellulose but makes no suggestion concerning antidandruff agent agglomerates.

It is a further object of this invention to not only provide effective antidandruff-depositing hair care compositions but also to provide said compositions with aesthetically-pleasing characteristics.

These and other objectives will become readily apparent from the detailed description that follows. Unless indicated to the contrary, all percentages and ratios are by weight.

## SUMMARY OF THE INVENTION

The present invention relates to antidandruff hair care compositions comprising from about 0.2% to about 30% of a surfactant, less than about 4.0% of a water insoluble, particulate antidandruff agent in agglomerate form wherein at least 20% of the agglomerates have an equivalent spherical diameter of

at least about 5 microns, and from about 50% to about 99% water, wherein said compositions are substantially free of clay and polymeric suspending agents.

DETAILED DESCRIPTION OF THE INVENTION

The hair care compositions of the present invention are comprised of certain essential components and may additionally contain several optional components. Each of these components is discussed in more detail below.

ANTIDANDRUFF AGENT

The water insoluble particulate antidandruff agent is present in agglomerated form in the compositions described herein at a level less than about 4.0%, preferably from about 0.1% to about 1.5%, most preferably from about 0.25% to about 1.25%. By water insoluble is meant having a solubility less than about 0.01% at 25°C.

The antidandruff agents useful herein can be any of a wide variety of materials including the previously mentioned zinc pyridinethione, sulfur and selenium sulfide. Other pyridinethione salts such as other heavy metal salts, tin, cadmium and zirconium, as well as the magnesium and aluminum salts may be used. Mixtures of agents may also be used. The preferred agent is zinc pyridinethione.

At least 20% of the agglomerates have an equivalent spherical diameter of at least 5 microns, preferably at least 7 microns, most preferably at least 10 microns. The method used to measure the particle size is x-ray sedimentation (Sedigraph 5000, Micromeritics Corp.). The particle sample is stirred prior to measurement rather than using more severe mixing techniques such as sonication. This allows the agglomerates to remain essentially intact and not split

into individual smaller particles. An advantage which agglomerates have over single, large particles, is that they can be made using conventional methods and equipment for forming the agent rather than the more involved processes associated with the latter. Processes for forming agglomerates are disclosed in Beddow, J.K., Particulate Science and Technology, Chemical Publishing Co., Inc. N.Y., N.Y. (1980), incorporated herein by reference.

SURFACTANTS

The surfactants useful in the present invention can be anionic, amphoteric, zwitterionic, nonionic, or cationic and are present at levels of from about 0.2% to about 30%, preferably from about 7% to about 20% for a shampoo and from about 0.2% to about 6.0% for use in creme rinses and conditioners. Cationic surfactants are not preferred in the shampoos of the present invention but are preferred for creme rinses which may contain the antidandruff agent.

Anionic surfactants can be exemplified by the alkali metal salts of organic sulfuric reaction products having in their molecular structure an alkyl radical containing from 8 - 22 carbon atoms and a sulfonic acid or sulfuric acid ester radical (included in the term alkyl is the alkyl portion of higher acyl radicals). Olefin sulfonates are included as suitable surfactants herein. Preferred are the sodium, ammonium, potassium or triethanolamine alkyl sulfates, especially those obtained by sulfating the higher alcohols ($C_8$ - $C_{18}$ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1

to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and others known in the art. Alkyl sulfates, ethoxylated alkyl sulfates and mixtures thereof are preferred. Particularly preferred are ammonium alkyl sulfate, triethanolamine alkyl sulfate, sodium alkyl sulfate, sodium alkyl glyceryl ether sulfonates and mixtures thereof.

Nonionic surfactants can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from about 6 to 12 carbon atoms in either a straight chain or branched chain configuration with ethylene oxide, the said ethylene oxide being present in amounts equal to 10 to 20 moles of ethylene oxide per mole of alkyl phenol. The alkyl substituent in such compounds may be derived from polymerized propylene, diisobutylene, octane, or nonane, for example.

2. The condensation product of aliphatic alcohols having from 8 to 18 carbon atoms, in either straight chain or branched chain configuration, with

ethylene oxide, e.g., a coconut alcohol ethylene oxide condensate having from 10 to 20 moles of ethylene oxide per mole of coconut alcohol, the coconut alcohol fraction having from 10 to 14 carbon atoms.

3. Long chain tertiary amine oxides corresponding to the following general formula:

$$R_1R_2R_3N \rightarrow O$$

wherein $R_1$ contains an alkyl, alkenyl or monohydroxy alkyl radical of from about 8 to about 18 carbon atoms, from 0 to about 10 ethylene oxide moieties, and from 0 to 1 glyceryl moiety, and $R_2$ and $R_3$ contain from 1 to about 3 carbon atoms and from 0 to about 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyldi(2-hydroxyethyl)amine oxide, dimethyloctylamine oxide, dimethyldecylamine oxide, dimethyltetradecylamine oxide, 3,6,9-trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.

4. Long chain tertiary phosphine oxides corresponding to the following general formula:

$$RR'R''P \rightarrow O$$

wherein R contains an alkyl, alkenyl or monohydroxyalkyl radical ranging from 8 to 18 carbon atoms in chain length, from 0 to about 10 ethylene oxide moieties and from 0 to 1 glyceryl moiety and R' and R" are each alkyl or monohydroxyalkyl groups containing from 1 to 3 carbon atoms. The arrow in the formula is

a conventional representation of a semipolar bond. Examples of suitable phosphine oxides are:

dodecyldimethylphosphine oxide,
tetradecyldimethylphosphine oxide,
tetradecylmethyldiethylphosphine oxide,
3,6,9-trioxaoctadecyldimethylphosphine oxide
cetyldimethylphosphine oxide,
3-dodecoxy-2-hydroxypropyldi-(2-hydroxyethyl)phosphine oxide,
stearyldimethylphosphine oxide,
cetylethylpropylphosphine oxide,
oleyldiethylphosphine oxide,
dodecyldiethylphosphine oxide,
tetradecyldiethylphosphine oxide,
dodecyldipropylphosphine oxide,
dodecyldi(hydroxymethyl)phosphine oxide,
dodecyldi(2-hydroxyethyl)phosphine oxide,
tetradecylmethyl-2-hydroxypropylphosphine oxide,
oleyldimethylphosphine oxide,
2-hydroxydodecyldimethylphosphine oxide.

5. Long chain dialkyl sulfoxides containing one short chain alkyl or hydroxy alkyl radical of 1 to about 3 carbon atoms (usually methyl) and one long hydrophobic chain which contain alkyl, alkenyl, hydroxy alkyl, or keto alkyl radicals containing from about 8 to about 20 carbon atoms, from 0 to about 10 ethylene oxide moieties and from 0 to 1 glyceryl moiety.

Examples include:

octadecyl methyl sulfoxide,
2-ketotridecyl methyl sulfoxide,
3,6,9-trioxaoctadecyl 2-hydroxyethyl sulfoxide,

dodecyl methyl sulfoxide,
oleyl 3-hydroxypropyl sulfoxide,
tetradecyl methyl sulfoxide,
3-methoxytridecyl methyl sulfoxide,
3-hydroxytridecyl methyl sulfoxide,
3-hydroxy-4-dodecoxybutyl methyl sulfoxide.

Zwitterionic surfactants can be exemplified by those which can be broadly described as derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

$$\begin{array}{c} \quad (R^3)_x \\ | \\ R^2 - Y^{(+)} - CH_2 - R^4 - Z^{(-)} \end{array}$$

wherein $R^2$ contains an alkyl, alkenyl, or hydroxy alkyl radical of from about 8 to about 18 carbon atoms, from 0 to about 10 ethylene oxide moieties and from 0 to 1 glyceryl moiety; Y is selected from the group consisting of nitrogen, phosphorus, and sulfur atoms; $R^3$ is an alkyl or monohydroxyalkyl group containing 1 to about 3 carbon atoms; X is 1 when Y is a sulfur atom and 2 when Y is a nitrogen or phosphorus atom; $R^4$ is an alkylene or hydroxyalkylene of from 1 to about 4 carbon atoms and Z is a radical selected from the group consisting of carboxylate, sulfonate, sulfate, phosphonate, and phosphate groups.

Examples include:

4-[N,N-di(2-hydroxyethyl)-N-octadecylammonio]-butane-1-carboxylate;

5-[S-3-hydroxypropyl-S-hexadecylsulfonio]-3-hydroxy-pentane-1-sulfate;
3-[P,P-diethyl-P-3,6,9-trioxatetradecoxylphosphonio]-2-hydroxypropane-1-phosphate;
3-[N,N-dipropyl-N-3-dodecoxy-2-hydroxypropylammonio]-propane-1-phosphonate;
3-(N,N-dimethyl-N-hexadecylammonio)propane-1-sulfonate;
3-(N,N-dimethyl-N-hexadecylammonio)-2-hydroxypropane-1-sulfonate;
4-[N,N]di(2-hydroxyethyl)-N-(2-hydroxydodecyl)ammonio]-butane-1-carboxylate;
3-[S-ethyl-S-(3-dodecoxy-2-hydroxypropyl)sulfonio]-propane-1-phosphate;
3-(P,P-dimethyl-P-dodecylphosphonio)-propane-1-phosphonate; and
5-[N,N-di(2-hydroxypropyl)-N-hexadecylammonio]-2-hydroxypentane-1-sulfate.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from 8 to about 18 carbon atoms and one contains an anionic water solubilizing group, e.g., carboxy, sulfonate, phosphate, or phosphonate. Examples of compounds falling within this definition are sodium 3-dodecylaminopropionate, sodium 3-dodecylaminopropane sulfonate, N-alkyltaurines such as the one prepared by reacting dodecylamine with sodium isethionate according to the teaching of U.S. Patent No. 2,658,072, N-higher alkyl aspartic acids such as those produced according to the teaching of U.S. Patent No.

2,438,091, and the products sold under the tradename "Miranol" and described in U.S. Patent No. 2,528,378.

Many cationic surfactants are known to the art. By way of example, the following may be mentioned:

dodecyltrimethylammonium chloride;
nonylbenzylethyldimethylammonium nitrate;
tetradecylpyridinium bromide;
laurylpyridinium chloride;
cetyltrimethylammonium chloride;
cetylpyridinium chloride;
laurylpyridinium chloride;
laurylisoquinolium bromide;
ditallow(hydrogenated)dimethyl ammonium chloride
dilauryldimethylammonium chloride; and
stearalkonium chloride

WATER

The last essential component of the compositions of the present invention is water. The amount of water can be from 50% to about 99%, preferably from about 75% to about 95%.

OPTIONAL COMPONENTS

Compositions of the present invention can contain any of a wide variety of optional components. Such optional components are more fully discussed below.

SUSPENDING AGENT

The suspending agents useful in the present compositions can be any of a number of non-polymeric, non-clay materials. Preferred materials include ethylene glycol esters of fatty acids having from about 16 to about 22 carbon atoms. Both ethylene glycol mono and distearate are examples of these esters.

Other useful suspending agents are alkanolamides of fatty acids having from about 16 to about 22 carbon

atoms, preferably about 16 to 18 carbon atoms. Suitable examples of these agents include stearic monoethanolamide, stearic diethanolamide, stearic monoisopropanolamide, stearic diethanolamide distearate and mixtures thereof.

Still other suitable suspending agents are alkyl ($C_{18}$ - $C_{22}$) dimethylamine oxides such as stearyl dimethyl amine oxide. Another acceptable suspending agent is a mixture of glyceryl stearate and stearamidoethyl diethylamine. Mixtures of suspending agents are also acceptable. When present, these suspending agents are at levels of from about 1% to about 7%, preferably from about 2% to about 6%. These agents may also serve to give an unexpected pearlescence to the product when used to suspend the antidandruff particles of the present invention. Smaller, unagglomerated particles tend to interfere with pearlescent agents.

AMIDE

An amide is a preferred optional component present in the shampoo aspect of the present invention.

Any of the alkanolamides of fatty acids useful in shampoo compositions can be used. Generally, these include mono and diethanolamides of fatty acids having from about 8 to about 14 carbon atoms. Preferred compounds include coconut monoethanolamide, lauric or coconut diethanolamide, and mixtures thereof.

The amide is present at a level of from about 2.0% to about 4.0%. It is believed that a more richer lathering, more stable product results when amides of these types are combined with the other components of the invention at hand.

FATTY ALCOHOLS

A desirable optional component when the compositions described herein are in the form of creme rinses/conditioners is a fatty alcohol such as cetyl alcohol, stearyl alcohol and mixtures thereof. These materials, if present, comprise from about 0.5% to about 5.0%, preferably from about 1.0% to about 3.0% of the total composition.

CONDITIONING AID

When the compositions of the present invention are in the form of shampoos, particularly where the surfactant is an anionic synthetic surfactant, a desirable optional component is a conditioning agent such as propylene glycol or cocoamidopropyl betaine at a level of from about 0.2% to about 5%. These materials surprisingly interfere less than other agents, such as fatty acids, with the deposition of the antidandruff agent.

OTHERS

Other ingredients useful in compositions of the present invention include preservatives such as benzyl alcohol, methyl paraben, propyl paraben and imidazolidinyl urea. In addition, pH adjusters such as citric acid, sodium citrate, succinic acid, phosphoric acid, sodium hydroxide, sodium carbonate, triethanolamine, and mixtures thereof may be useful in the present invention. Emulsifiers may be useful for forming stable creme rinse/conditioner products. Other optional ingredients such as perfumes, dyes and coloring agents may also be added. These aesthetic agents as well as the others comprise, individually, form about 0.1% to about 2% of the composition if they are present.

The pH of the present compositions ranges from about 3 to about 9, preferably from about 3 to about

6. Proper pH is obtained through the use of an appropriate buffer system such as those described above. Other buffering systems are known to those knowledgeable in the art.

The compositions of the present invention are substantially free of polymeric and clay type suspending agents. Such agents include, for example, carboxymethylcellulose, hydroxypropyl guar gum, hydroxyethylcellulose, acrylic acid polymers, magnesium aluminum silicate, hydroxypropylmethylcellulose. It is believed that these suspending agents interfere with the formation of a final product which possesses efficient antidandruff agent deposition. These should not be added as a separate component or with other materials such as the antidandruff agent. By substantially free is meant less than about 0.02%. Not included as polymeric suspending agents herein are surfactants which may be polymeric.

METHOD OF MANUFACTURE

The antidandruff hair care compositions of the present invention may be made by mixing some or all of the ingredients except the antidandruff agent, but including agents like the suspending agent and amide, at temperatures ranging from about 71°C. to about 88°C., cooling this mixture to from about 25°C. to about 49°C., and adding the remaining ingredients including the antidandruff agent to the cooled mixture.

INDUSTRIAL APPLICABILITY

The antidandruff compositions of the present invention are used in a conventional manner. Shampoos being used in an amount of about 10 grams while creme rinses are used in an amount of from about 5 grams to about 15 grams. The following examples are provided

to further describe and demonstrate embodiments of the present invention. These examples are merely illustrative of the present invention and are not limitative thereof.

EXAMPLE I

The following shampoo composition of the present invention was prepared:

| Component | Weight % |
|---|---|
| ZPT (48.5% Active)* | 2.07 |
| Triethanolamine Alkyl Sulfate (35% Active) | 55.56 |
| NaCl | 0.70 |
| Citric Acid | 0.65 |
| Coconut Monoethanolamide | 3.00 |
| Ethylene Glycol Distearate | 5.00 |
| Perfume | 0.60 |
| Color solution | 0.32 |
| Water | q.s. 100% |

*Agglomerates wherein 20% have an equivalent spherical diameter of at least 5 microns.

EXAMPLE II

A composition identical with that of Example I was prepared except that 20% of the ZPT particles had equivalent spherical diameters of at least 7 microns.

EXAMPLE III

An antidandruff shampoo composition of the present invention is prepared with the following components:

| Component | Weight % |
|---|---|
| ZPT (48% Active)* | 3.13 |
| Sodium Alkyl Sulfate (28% Active) | 44.30 |
| Ammonium Alkyl Sulfate (25% Active) | 11.10 |
| Citric Acid | 0.45 |
| Ammonium Citrate | 0.30 |
| Ammonium Xylene Sulfonate | 1.00 |
| Coconut Monoethanolamide | 2.50 |
| Ethylene Glycol Distearate | 5.00 |
| Perfume | 0.60 |
| Color solution | 0.16 |
| Water | q.s. 100% |

* In this and the following Examples the ZPT is that of Example I.

EXAMPLE IV

The following composition is prepared:

| Component | Weight % |
|---|---|
| ZPT (48% Active)* | 2.08 |
| Sodium Alkyl Sulfate (28% Active) | 20.00 |
| Ammonium Alkyl Sulfate (28% Active) | 35.00 |
| Ammonium Chloride | 0.60 |
| Propylene Glycol | 3.85 |
| Citric Acid | 0.35 |
| Ammonium Citrate | 0.40 |
| Coconut Monoethanolamide | 3.00 |
| Ethylene Glycol Distearate | 5.00 |
| Perfume | 0.60 |
| Color solution | 0.16 |
| Water | q.s. 100% |

EXAMPLE V

The following hair care, creme rinse composition containing cationic surfactants is prepared:

| Component | Weight % |
|---|---|
| ZPT (48% Active)* | 1.04 |
| Stearalkonium Chloride | 1.60 |
| Cetyl Trimethylammonium Chloride | 1.60 |
| Cetyl Alcohol | 1.35 |
| Stearyl Alcohol | 1.35 |
| Ceteth-2 | 0.80 |
| Crotein Q[1] | 0.50 |
| Citric Acid | 0.11 |
| Sodium Chloride | 0.10 |
| Perfume | 0.20 |
| Color solution | 0.06 |
| Kathon CG[2] | 0.033 |
| Water | q.s. 100% |

1 A quaternized protein offered by Croda, Inc.

2 Preservative offered by Rohm and Haas.

WHAT IS CLAIMED IS:

Claims

1. An antidandruff hair care composition characterised by:
   (a) a water insoluble, particulate antidandruff agent present at a level of less than 4%, said agent being in the form of agglomerates wherein at least 20% of said agglomerates have an equivalent spherical diameter of at least 5 microns;
   (b) from 0.2% to 30% surfactant; and
   (c) water,

   wherein said composition is substantially free of polymeric and clay type suspending agents.

2. An antidandruff hair care composition according to Claim 1 which is in the form of a shampoo and wherein the level of surfactant is from 7% to 20% and the antidandruff agent is present at a level of from 0.1% to 1.5%.

3. An antidandruff shampoo composition according to Claim 1 or 2 characterised in that the surfactant is an anionic surfactant selected from alkyl sulfates, ethoxylated alkyl sulfates, sodium alkyl glyceryl ether sulfonates, and mixtures thereof.

4. An antidandruff shampoo composition according to any of Claims 1 to 3 characterised in that the antidandruff agent is selected from sulfur, selenium sulfide, heavy metal, magnesium and aluminum pyridinethione salts and mixtures thereof.

5. An antidandruff shampoo composition according to any of Claims 1 to 4 characterised in addition by an alkanol amide of a fatty acid containing from 8 to 14 carbon atoms and a nonclay, nonpolymeric suspending agent.

6. An antidandruff shampoo composition according to Claim 5 characterised in that the suspending agent is selected from ethylene glycol monostearate, ethylene glycol distearate, and mixtures thereof.

7. An antidandruff shampoo composition according to Claim 5 or 6 characterised in that the alkanolamide of a fatty acid is selected from coconut monoethanolamide, lauric or coconut diethanolamide and mixtures thereof.

8. An antidandruff shampoo composition according to any of Claims 1 to 7 characterised in that the surfactant is selected from sodium alkyl sulfate, triethanolamine alkyl sulfate, ammonium alkyl sulfate and mixtures thereof.

9. An antidandruff shampoo composition according to any of Claims 1 to 8 characterised in that the water insoluble, particulate antidandruff agent is zinc pyridinethione.

10. An antidandruff shampoo composition according to any of Claims 1 to 9 characterised in addition by from 0.2% to 5% of a conditioning aid selected from propylene glycol and cocoamidopropyl betaine.

11. An antidandruff hair care composition according to Claim 1 in the form of a creme rinse wherein the surfactant is cationic.

12. An antidandruff creme rinse composition according to Claim 11 wherein the level of surfactant is from 0.5% to 4.0%.

13. An antidandruff creme rinse composition according to Claim 11 or 12 wherein additionally a fatty alcohol is present at a level of from 1.0% to 3.0%.

14. An antidandruff creme rinse composition according to Claim 13 wherein the fatty alcohol is selected from cetyl alcohol, stearyl alcohol and mixtures thereof.